(19) **Europäisches Patentamt**

**European Patent Office**

**Office européen des brevets**

(11) **EP 1 061 368 B1**

(12) **EUROPEAN PATENT SPECIFICATION**

(45) Date of publication and mention
of the grant of the patent:
**23.03.2005 Bulletin 2005/12**

(51) Int Cl.⁷: **G01N 33/53**, G01N 33/539,
G01N 33/553, G01N 33/52
// G01N33/72

(21) Application number: **00111959.3**

(22) Date of filing: **16.06.2000**

(54) **Agglutination assay method in binder medium**

Agglutinations-Bestimmungsmethode in einem Bindermedium

Essai d'agglutination dans un milieu liant

(84) Designated Contracting States:
**DE FR GB**

(30) Priority: **18.06.1999 JP 17249499**

(43) Date of publication of application:
**20.12.2000 Bulletin 2000/51**

(73) Proprietor: **FUJI PHOTO FILM CO., LTD.
Kanagawa 250-01 (JP)**

(72) Inventors:
• **Kawasaki, Kazuya
Asaka-shi, Saitama (JP)**
• **Nakamura, Kentaro
Asaka-shi, Saitama (JP)**
• **Seshimoto, Osamu
Asaka-shi, Saitama (JP)**
• **Nagata, Masahito
Tokyo (JP)**
• **Tanaka, Toru
Tokyo (JP)**

(74) Representative: **Albrecht, Thomas, Dr. et al
Kraus & Weisert
Patent- und Rechtsanwälte
Thomas-Wimmer-Ring 15
80539 München (DE)**

(56) References cited:
EP-A1- 0 252 750      EP-A1- 0 524 596
EP-A2- 0 258 963      US-A- 4 283 491

**Description**

The present invention relates to a method for detecting and analyzing a trace substance by utilizing the agglutination assay, in which an analyte reacts with a particle-labeled anti-analyte, such as an antibody, to cause the particle agglutination. Particularly, the present invention relates to a dry analysis method for causing the agglutination of the particles bearing the anti-analyte in a layer construction of a dry analysis element. Also, the present invention relates to a dry analysis element which enables such analysis method.

BACKGROUND OF THE INVENTION

In recent years, it has come to be very important to quantitatively analyze a trace substance, particularly antibody or antigen, in a specimen promptly, conveniently and precisely in order to diagnose the condition of diseases or judge the effects of treatment. For this purpose, widely employed has been an immuno-serological test for assaying the existence of an antigen or antibody in the body fluid, in which the antibody or antigen is adsorbed and immobilized to insoluble carrier particles and the resulting particles are reacted with the antigen or antibody.

The latex particles agglutination immunoassay is performed routinely by mixing a suspension of antibody-coated latex particles (sensitized latex) with a specimen on a glass plate. The latex particles agglutinate, or fail to agglutinate, as a result of interacting with the analyte antigen in the specimen. The extent of the agglutination can be determined by visual inspection. This assay makes it possible to semi-quantitatively analyze the antigen in the specimen by diluting the specimen at various ratios similar to another qualitative assay.

In Japanese Patent Publication Nos. 11575/1983, 43138/1987 and 55013/1987, there is proposed a method, in which latex particles having an antibody bound thereto is reacted with the antigen in the sample and the amount of the agglutination of the latex particles is determined optically by nephelometry. According to the proposed method, an antigen or antibody has come to be analyzed quantitatively by an automatic analyzer.

In addition, Unexamined Japanese Patent Publication (KOKAI) Nos. 141665/1990, 94719/1994 and 213891/1994 disclose a method wherein an antigenic substance is detected by measuring a change in the absorbance upon the agglutination of the colloidal gold-labeled antibodies.

The above-described immunoassays do not require B/F separation and in this point, they are useful. The latex reagent is, however, poor in storage stability, since it is in the liquid form. In the colloidal gold agglutination, the colloidal gold solution or dispersion is not suitable as a reagent because of poor storage stability. A colloidal gold-labeled reagent in the lyophilized form must be mixed with a dedicated solution upon measurement, which makes the operation cumbersome. This method is also accompanied with such a drawback as unsuitability for use in the measurement of a small amount of a sample.

A so-called dry analysis method is, on the other hand, superior in storage stability and convenient operation. The so-called wet system (or solution system) comprises dissolving a reagent to be used for the assay in an aqueous solvent, thereby preparing the corresponding reagent solution, adding this reagent solution to a sample to be analyzed and then measuring the color reaction product by a colorimeter. On the other hand, the dry analysis method comprises spotting an aqueous sample directly to a dry analysis element, such as test piece, analytical slide or analytical tape, having a reagent composition incorporated therein in the dry form and effecting colorimetry of the color development or color change occurring in the element. The dry system is superior to the wet system using a reagent solution in convenient operation and speedy assay.

A method for causing agglutination in the layer of a dry analysis element, thereby directly detecting the existence of an agglutinate itself in the layer construction has not yet been proposed.

A dry analysis method, so-called solid phase immunochromatography method has also been proposed (for example, Unexamined Japanese Patent Publication (KOKAI) No. 5326/1997, which corresponds EP 0834741A1). This method utilizes a chromatographic medium which is a liquid-permeable material serving a capillary action. The liquid-permeable sheet such as filter paper has a sample feeding zone and a detection zone, the sample feeding zone containing an colloidal gold-labeled antibody, and the detection zone containing an immobilized second antibody for binding to the different epitope of the analyte antigen. The second antibody is used as a capturing antibody. When a test sample containing an analyte antigen is fed to the sample feeding zone containing the colloidal gold-labeled antibody, the analyte antigen reacts with the colloidal gold-labeled antibody to form an immunocomplex. The formed complex diffuses and migrates to the detection zone containing the immobilized second antibody, by the capillary action of the chromatographic medium. The complex of the antigen and colloidal gold-labeled antibody are captured by the immobilized second antibody. The existence of the analyte antigen is confirmed by detecting the color tone of the colloidal gold which appears in the detection zone containing the capturing second antibody. Since the reagent used in this method is maintained at dry condition just before assay, it is excellent in storage stability. However, it is a problem that the result is not quantitative. Furthermore, in principle, the method is a sandwich method in which a colloidal gold-labeled antibody is captured by a second antibody through intervening analyte antigen, it is necessary to use the permeable medium sheet having a sufficiently large area so that an excessive colloidal gold-labeled antibody is diffused and removed from the detection zone to which the capturing second antibody is immobilized. This is the reason why the method is called as immunochromatography method. Accordingly, a plenty of liquid must be fed to the sheet, and it is necessary to use a large medium sheet. In addition, the immunochromatography method requires a long assay time, since it takes enough time for removal of an excessive colloidal gold-labeled antibody from the capturing zone by the capillary action.

Under such a circumstance, the present inventors have attempted to search for a material capable of causing agglutination in a layer medium of a dry analysis element. As a result, agglutination of a colloidal metal in an analysis element can be caused quantitatively at good sensitivity by using some kind of medium, and storage stability of the reagent, which is an important characteristic of a dry analysis element, is also successfully attained.

SUMMARY OF THE INVENTION

The present invention has been accomplished in view of the aforementioned circumstances, and a first object thereof is to provide a dry analysis method for determining an analyte using an agglutination of the particles bearing an anti-analyte, by which a high sensitive analysis is ensured while using a simple operation and reagent can be stored with excellent stability in the dry state.

A second object of the present invention is to provide a dry analysis element which can detect agglutination caused by the reaction between an analyte and an anti-analyte labeled with labeling particle, thereby analyzing the analyte in a convenient and highly sensitive manner.

The first object of the present invention is attained by an agglutination assay method for quantitative determination of an analyte in an aqueous liquid sample using particles bearing an anti-analyte, the anti-analyte being capable of specifically binding to the analyte so as to cause agglutination of the particles, comprising:

providing a reagent layer composed of at least one binder selected from the group consisting of:

1) a water-soluble polymer, a solution of which has a viscosity of 6 cP or less;
2) a water-insoluble and water-swellable polymer; and
3) gelatin having a molecular weight of 20,000 or less;

supplying said sample, together with said particles, to said reagent layer to cause the agglutination of said particles in said reagent layer; and
measuring the extent of the agglutination of the particles in the reagent layer to determine the amount of the analyte in the sample.

In the present invention, the agglutination of particles bearing an anti-analyte (such as a colloidal metal-labeled antibody) (which is also referred to as labeling particle or carrier) is conducted in a reagent layer composed of a binder medium comprising any of a water-soluble polymer having a solution viscosity of 6 cP or less, a water-insoluble and water-swellable polymer, and gelatin having a molecular weight of 20,000 or less. By employing these binder mediums, the reagent layer can be made dry state to an extent not harmful to the stability of the reagent composition to be used upon storage. While upon analysis, the reagent layer is wetted with an aqueous test sample and thereby acquires fluidity sufficient for causing agglutination of labeling particles bearing an anti-analyte.

The labeling particles may be fed together with an analyte upon assay. Alternatively, the particles bearing an anti-analyte is incorporated in the reagent layer in advance, and the particles bearing an anti-analyte may be subjected to agglutination by the immunoreaction with an analyte.

The extent of the agglutination caused in the reagent layer is easily detected by measuring an optical change of the transmitted or reflected light from outside of the reagent layer. The existence of the agglutinate and its amount may be detected as a turbidity change in the reagent layer medium, or as a change in color tone of the labeling particle due to agglutination.

The second object of the present invention is attained by a dry analysis element for quantitative determination of an analyte in an aqueous liquid sample by measuring the extent of agglutination of particles bearing an anti-analyte, the anti-analyte being capable of specifically binding to the analyte so as to cause agglutination of the particles, comprising:

a reagent layer composed of at least one binder selected from the group consisting of:

1) a water-soluble polymer, a solution of which has a viscosity of 6 cP or less;
2) a water-insoluble and water-swellable polymer; and

3) gelatin having a molecular weight of 20,000 or less;

whereby, when the sample is applied to the reagent layer together with said particles, the agglutination of said particles takes place in the reagent layer.

In a preferred embodiment, the reagent layer contains particles bearing an anti-analyte. Furthermore, a spreading layer may be laminated on the reagent layer. In this case, the spreading layer may contain particles bearing an anti-analyte so as to transfer the particles bearing an anti-analyte together with an analyte into the reagent layer when an aqueous sample solution is spotted.

BRIEF DESCRIPTION OF THE DRAWINGS

Fig. 1 is an illustration showing the layer structure of one embodiment of the dry analysis element according to the present invention;
Fig. 2 is an illustration showing the layer structure of second embodiment of the dry analysis element according to the present invention;
Fig. 3 is a graphic representation showing the results of Example 4, more specifically, calibration curves of dry analysis elements of the slide 1 obtained in the Example 4;
Fig. 4 is a graphic representation showing the results of Example 5, more specifically, calibration curves of dry analysis elements of the slide 2 obtained in the Example 5; and
Fig. 5 is a graphic representation showing the results of Example 6, more specifically, calibration curves of dry analysis elements of the slide 3 obtained in the Example 6.

DETAILED DESCRIPTION OF THE INVENTION

Analyte and Anti-Analyte

As an analyte or a substance to be analyzed in the present invention, any substance can be used insofar as there exists a specific binding partner or substance thereto in the nature or such a substance can be prepared by chemical means. The anti-analyte, i.e., specific binding partner or substance as used herein means a substance which can specifically recognizes and binds to the analyte and at the same time, can be bound to a labeling carrier particle.

Examples of the combination of an analyte and an anti-analyte thereto include combinations of antigen and antibody, a certain saccharide and lectin, biotin and avidin, protein A and IgG, hormone and receptor thereof, enzyme and substrate, and nucleic acid and complementary nucleic acid. In the above-exemplified combinations, the analyte and the anti-analyte may be reversed.

The most ordinary example is a combination of an antigen as an analyte and an antibody as an anti-analyte. The antibody as an anti-analyte may be either a polyclonal or monoclonal antibody. Alternatively, a plurality of different antibodies can be used. No particular limitation is imposed on the class of the antibody and it does not matter whether it is IgG or IgM. It may be a fragment of an antibody, for example, Fab, Fab' or $F(ab')_2$. When a monoclonal antibody is employed as a specific binding substance, an analyte antigen must have at least two same epitopes in order to cause agglutination of a labeling particle having an antibody bound thereon. Alternatively, at least two different antibodies which binds to different epitopes of the analyte antigen, respectively, may be bound to the labeling carrier particle. When the analyte antigen is composed of plural sub-units, such as hemoglobin, however, there is no need to use plural different monoclonal antibodies. Binding plural molecules of single kind of monoclonal antibody to the labeling carrier (particle), the agglutination of the particles can be caused by the reaction with the analyte antigen. At least two antibody molecules are preferably bound to the labeling carrier (particle) for causing agglutination.

Labeling Particle

As a labeling carrier or particle for labeling by binding an anti-analyte, any particle can be used insofar as it undergoes agglutination as a result of reaction with the analyte and the anti-analyte bound to the particle and the extent of the agglutination falls within a detectable range. As the labeling particle, those ordinarily employed for immuno-agglutination can be used. Examples of the carrier particle include organic high-molecular latex particles such as polystyrene or styrene-butadiene copolymer, and metals such as colloidal metal. The labeling particles (or colloid) are preferred to have an average particle size falling within a range of 0.02 to 10 μm. When the particles have an excessively large particle size, optical strength due to optical reflection or light scattering of the particle itself prior to the immunoreaction becomes too high, resulting in difficulty in measurement of the change of the optical density. Too small particle sizes, on the other hand, tend to lower the detection sensitivity of the agglutinate.

Any conventionally known colloidal metal can be used as a labeling particle. Examples include colloidal gold, colloidal silver, colloidal platinum, colloidal iron and colloidal aluminum hydroxide. In particular, colloidal gold and colloidal silver are preferred because they colors red and yellow, respectively, at a proper particle size. The particle size of a colloidal metal is preferably about 1 to 500 nm. The size of 5 to 100 nm is particularly preferred, because it permits development of a strong color tone.

The colloidal metal and the anti-analyte can be bound in a conventionally known manner (for example, The Journal of Histochemistry and Cytochemistry, Vol.30, No.7, pp 691-696 (1982)). For example, a colloidal metal and an anti-analyte (i.g., an antibody) are mixed in a proper buffer solution and incubated at room temperature for at least 5 minutes. The reaction mixture is centrifuged to remove a supernatant. The obtained precipitate is dispersed into a solution containing a dispersant such as polyethylene glycol to obtain an aimed colloidal metal bearing an anti-analyte.

In the case of using a colloidal gold particle as the colloidal metal, commercially available one may be employed. Alternatively, a colloidal gold particle can be prepared according to a conventional method, for example, a method of reducing chloroauric acid with sodium citrate (Nature Phys. Sci., Vol.241, 20(1973) etc.).

Binder (Medium of the Reagent Layer)

As the binder composing the reagent layer, any of the following can be employed alone or in combination:

> 1) a water-soluble polymer, a solution of which has a viscosity of 6 cP or less;
> 2) a water-insoluble and water-swellable polymer; and
> 3) gelatin having a molecular weight of 20,000 or less.

As shown in the working examples described hereinafter, upon spotting a sample solution, agglutination of labeling particles can be caused quantitatively in the reagent layer where these binders are employed as medium.

Examples of the water-soluble polymer having a solution viscosity of 6 cP or less include acrylamide-N-vinylpyrrolidone copolymer;

acrylamide-N-vinylpyrrolidone-methacryl alcohol copolymer such as $(acrylamide)_{50}$-$(N\text{-vinylpyrrolidone})_{38}$-$(methacryl\ alcohol)_2$;

polyvinylpyrrolidone ($[C_6H_9NO\text{-}]_n$);

polyacrylamide ($[\text{-}CH_2CH(CONH_2)\text{-}]_n$);

$(CH_2CH\text{-}COOCH_2CH(OH)CH_3)_{60}$-$(CH_2CH\text{-}CONHCCH_2SO_3(CH_3)_2)_{40}$ and the like.

In the meantimes, the viscosity of polymer solution defined as "6 cP" is the one measured as follows: said polymer is dissolved in a 50 mM sodium citrate solution (pH 6.0) containing 0.1% sodium azide and 0.01% Triton X-100, to prepare 2% polymer solution and the viscosity of the 2% polymer solution is measured at 40°C by means of B-type viscometer (Brookfield type viscometer).

In addition, examples of the water-insoluble and water-swellable polymer include a water-insoluble starch such as carboxymethylated starch, and carboxymethyl cellulose.

Layer Structure of Dry Analysis Element

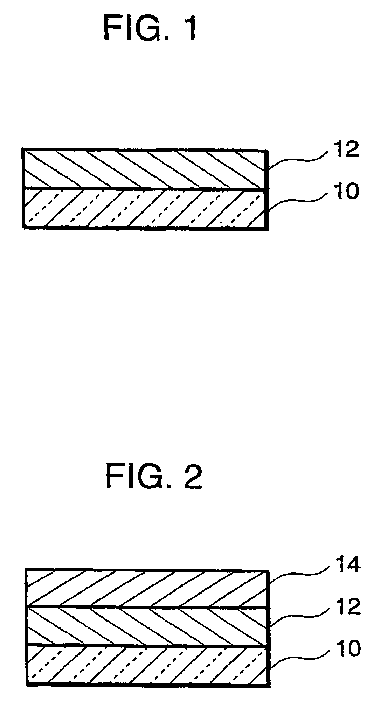

Fig. 1 shows the layer structure of an embodiment of the dry analysis element according to the invention. In Fig. 1, reference numeral 10 designates a support on which a reagent layer 12 is laminated.

The support 10 may be light non-transmitting (opaque), light-semi-transmitting (translucent), or light-transmitting (transparent), and it is generally preferable that the support is light-transmitting and water-impermeable. Preferable materials for the light-transmitting and water-impermeable support are polyethylene terephthalate, polystyrene or the like. In general, an undercoating is provided or the support is subjected to hydrophilization treatment in order to firmly adhere the reagent layer to be laminated thereon.

The reagent layer 12 is a layer where aforementioned binder(s) is used as medium, and is a reaction layer in which agglutination of labeling particles is caused by the immunoreaction between the analyte and the anti-analyte bound to the particle.

In the reagent layer 12, a buffer may be incorporated so that the specific binding reaction between the particle-labeled anti-analyte and the analyte occurs at an optimum pH. For the antigen-antibody reaction, for example, pH buffers usable for ordinary antigen-antibody reaction can be employed. Specific examples of usable buffers are buffer reagents containing tris(hydroxymethyl)aminomethane (Tris), buffer reagents containing phosphate, buffer reagents containing borate, buffer reagents containing citric acid or citrate, buffer reagents containing glycine, buffer reagents containing Bicine, buffer reagents containing HEPES, and buffer reagents containing Good's buffer agent such as MES (2-morpholinoethanesulfonic acid). The reaction may be effected at any pH insofar as the pH is within a range permitting ordinary antigen-antibody reaction.

In the reagent layer, a high molecular polymer such as polyvinyl alcohol, polyvinyl pyrrolidone or PEG (polyethylene glycol) may be incorporated for the purpose of promoting agglutination.

The reagent layer may be provided by coating an aqueous solution or dispersion containing the aforementioned binder and additional other reagent composition on another layer, such as a support or a detecting layer, and then drying the coated solution or dispersion, as disclosed in the specifications of Japanese Patent Publication No. 21677/1978 (corresponding to USP 3,992,158), Unexamined Japanese Patent Publication (KOKAI) Nos. 164356/1980 (corresponding to USP 4,292,272), 101398/1979 (corresponding to USP 4,132,528), 292063/1986 (Chemical Abstracts, 106; 210567y). The thickness of the dried reagent layer containing aforementioned binder may range from about 2 μm to about 50 μm, and preferably, from about 4 μm to about 30 μm, and the coverage thereof may range from about 2 g/m$^2$ to about 50 g/m$^2$, and preferably, from about 4 g/m$^2$ to about 30 g/m$^2$.

The reagent layer 12 may contain particles bearing an anti-analyte in advance. In this case, agglutination can be caused in the reagent layer 12 by simply applying a liquid sample containing an analyte to the reagent layer 12.

Fig. 2 shows second embodiment of dry analysis element according to the present invention. In this embodiment, a spreading layer 14 is further laminated on the reagent layer 12. The spreading layer is a layer having a so-called metering function to spread a liquid over an area substantially in proportion to the volume of the liquid fed thereto. The existence of the spreading layer 14 makes an amount of the liquid fed to the reagent layer 12 per area uniformly and thereby accuracy at quantitative determination of the analyte by agglutination in the reagent layer 12 is improved.

The spreading layer is preferably a porous layer and may be fibrous or non-fibrous. As the fibrous material, filter paper, non-woven cloth, woven cloth (e.g., plain woven cloth such as broad and poplin), knitted cloth (e.g., knitted cloth such as tricot, double tricot, and milaneaze) or filter paper made of glass fibers may be used. Examples of the non-fibrous material may be either one of a membrane filter composed of cellulose acetate as described in Unexamined Japanese Patent Publication (KOKAI) No. 53888/1974 (corresponding to USP 3,992,258), or a particulate structure layer containing interconnected voids and composed of inorganic or organic fine particles as disclosed in Unexamined Japanese Patent Publication (KOKAI) Nos. 53888/1974 (corresponding to USP 3,992,258), 90859/1980 (corresponding to USP 4,258,001) and 70163/1983 (corresponding to USP 4,486,537). A laminated structure made of partially bonded multiple porous layers may also be preferably used, examples of such structure being disclosed in Unexamined Japanese Patent Publication (KOKAI) Nos. 4959/1986 (corresponding to EP 0166365A), 116248/1987, 138756/1987 (corresponding to EP 0226465A), 138757/1987 (corresponding to EP 0226465A) and 138758/1987 (corresponding to EP 0226465A).

Preferable materials for the spreading layer are woven and knitted fabrics. The woven fabrics or like may be subjected to the glow discharge treatment as described in Unexamined Japanese Patent Publication (KOKAI) No. 663599/1982 (corresponding to USP 4,783,315 and GB 2,087,974A). In order to adjust the area or rate for spreading, the spreading layer may contain a hydrophilic polymer or a surfactant as described in Unexamined Japanese Patent Publication (KOKAI) Nos. 222770/1985 (corresponding to EP 0162301A), 219397/1988 (corresponding to DE 37 17 913A), 112999/1988 (corresponding to DE 37 17 913A) and 182652/1987 (corresponding to DE 37 17 913A).

In addition, the spreading layer 14 may contain light reflecting fine particles of, for example, titanium dioxide or barium sulfate so as to serve a light reflecting function. The spreading layer 14 having the light reflecting or light shielding function may act as a white background so that change of color or color density caused by agglutination in the reagent layer 12 is reflectively measured from the light-transmitting support 10 side. When the spreading layer 14 itself possesses an optical property suitable for white background, the layer may not contain light-reflecting fine particles.

Instead of the reagent layer 12, the spreading layer 14 may contain the labeling particles bearing an anti-analyte. In this case, by spotting to apply an aqueous test sample onto the reagent layer 14, the labeling particles in the spreading layer 14 can be transferred together with an analyte into the reagent layer 12 to cause agglutination in the reagent layer 12.

Preparation of Dry Analysis Element

The dry analysis element of the invention may be prepared by any of the known processes described in the specifications of the aforequoted patents. The analysis element of the invention may be cut into a square piece having sides each ranging from about 15 to 30 mm or a disk having a substantially same area. It is preferred, in view of the preparation, packaging, shipping, storage and measuring operations, that the element be contained in a slide frame as descried, for example, in Japanese Patent Publication No. 28331/1982 (corresponding to USP 4,169,751), Unexamined Japanese Utility Model Publication No. 142454/1981 (corresponding to USP 4,387,990), Unexamined Japanese Patent Publication No. 63452/1982, Unexamined Japanese Utility Model publication No. 32350/1983 and Unexamined Japanese Patent publication No. 501144/1983 (corresponding to International Publication WO 83/00391) for use as a slide for chemical analysis. For the convenience in some uses, it may be formed in a long tape shape which is contained in a cassette or magazine, or a small piece thereof may be applied on or contained in a card having an opening.

Analysis Method Using the Dry Analysis Element

The analysis element of the invention may be used for the quantitative analysis of an analyte in a sample liquid by using it through the operations described in the specifications of the aforequoted patents. When the analyte is an antigen or an antibody, about 5 µL to about 30 µL, preferably 8 µL to 15 µL, of an aqueous sample liquid such as plasma, serum or urine is spotted on the reagent layer 12 or, in the case that the spreading layer 14 is laminated thereon, on the spreading layer 14. The analysis element thus spotted is then incubated at a constant temperature of from about 20°C to about 45°C, preferably at a constant temperature of from about 30°C to about 40°C, for 1 to 10 minutes. The reflection optical density of the color or the change in color in the element may be measured from the light-transmitting support side, and the quantity of the analyte contained in the sample can be determined using a preliminarily prepared calibration curve based on the principle of colorimetry. The volume of the spotted liquid sample and the time and temperature for incubation are maintained constant to improve the accuracy in quantitative analysis.

The measuring operation may be carried out while using the chemical analysis apparatuses described in Unexamined Japanese Patent Publication Nos. 125543/1985, 220862/1985, 294367/1986 and 161867/1983 (corresponding to USP 4,424,191) to realize quantitative analysis at a high accuracy by extremely easy operation. Depending on the purpose or required precision, however, semi-quantitative measurement may be conducted by visually judging the degree of coloring or change of color tone.

When the analysis element does not contain the labeling particles bearing an anti-analyte, a necessary immunological reaction can be carried out in a proper reaction mixture other than the element, and then the resultant reaction mixture is spotted on the element. Thus the analyte can be analyzed. For assaying an antigen, for example, an aqueous sample liquid is mixed with a solution containing an antibody labeled with the labeling particle to complete the binding reaction, and then spotted on the element.

For example, when an antigen, an antibody, a colloidal metal, and carboxymethylated starch are used as an analyte, an anti-analyte, a labeling carrier particle, and a binder for medium of the reagent layer, respectively, a dry analysis element can be prepared and a dry analysis using the element can be carried out as described below.

Specifically, an antibody labeled with a colloidal metal is dispersed in a solution of carboxymethylated starch. The resulting dispersion is applied to a light-transmitting support 10, followed by drying, whereby a reagent layer 12 is formed and thus a dry analysis element for agglutination assay can be prepared.

An aqueous liquid sample containing an analyte (e,g., antigen) is spotted and applied onto the resulting analysis element. The analyte antigen causes the antigen-antibody binding reaction with the colloidal metal-labeled antibody in the carboxymethylated starch layer 12, resulting in agglutination of the colloidal metal.

Agglutination changes the color tone or hue of the colloidal metal so that the analyte in the sample can be detected and quantitatively analyzed by measuring a change in the color tone of the reagent layer. For example, a colloidal gold before agglutination colors reddish violet having a main absorption wavelength at about 540 nm. By the agglutination, the colloidal gold increases in size, leading to shifting of its absorbance to the side of a longer wavelength, and as a result, the agglutinated colloidal gold colors pale reddish purple or gray. Accordingly, the analyte (antigen) can be quantitatively analyzed from a decrease in the reflection optical density at 540 nm, an increase in the reflection optical density at about 630 nm which appears by agglutination, or a difference between reflection optical densities at 540 nm and 630 nm.

Example 1

Selection of Water-Soluble Polymer to which Colloidal Metal is incorporated

Experiments of agglutination in solution system were conducted as described below while using an immunological kit for detecting fecal occult hemoglobin "Immuno-Gold Hem" (produced by Godo Shusei Co., Ltd., sold by Wako Pure Chemicals Industries Ltd.). Various polymers were added to the reaction system so as to make the final concentration to be 1.6% by weight. As described in Example 4 mentioned below, in the case that a reagent layer was prepared with using a water-soluble polymer as binder, usually around 3% solution of the polymer was coated on a support, whereby the coverage of the polymer being about 7.5 g/m$^2$. When about 10 µL of an aqueous sample was spotted on the reagent layer having such coverage of the polymer, the aqueous sample spreads in the reagent layer to result in a disc shape having a diameter of about 5 mm. From calculation based on the liquid amount at spotting and the spread area, the polymer concentration in the reagent layer is about 1.5% at the area where the aqueous sample is applied. Regarding such a final concentration of the polymer in the analysis element, experiments for selecting polymers suppressing no agglutination in the analysis element were herein conducted while adding various polymers to the reaction system so that the final concentration was made to be 1.6% by weight.

According to the direction for use of the kit, one bottle of a colloidal gold antibody reagent (containing 2 mg of conjugate of colloidal gold and mouse monoclonal anti-human hemoglobin antibody) was dissolved in 2.5 mL of a liquid for dissolving colloidal gold reagent contained in the kit to prepare a colloidal gold-labeled antibody solution. At that time, each of various water-soluble polymers was added to the colloidal gold-labeled antibody solution so as to make the concentration to be 2.5%.

Human hemoglobin $A_0$ (product of Exocell INC.) was diluted with an aqueous solution of 0.2M ammonium chloride (pH 6.8) containing 6% polyethylene glycol 6000 to prepare 0 ng/mL and 1000 ng/mL of hemoglobin (Hb) solutions, which were used as sample liquids hereinafter. While using the colloidal gold-labeled antibody solution to which each of various polymers was added, the Hb sample liquid was assayed by following to the direction of the kit. Namely, one drop (50 μL) of the Hb sample liquid was added to a well of a microtiter plate, to which two drops (90 μL) of a colloidal gold-labeled antibody solution containing each of various polymers was then added, followed by reacting at room temperature for 5 minutes after gentle mixing. The polymer concentration in the agglutination system was calculated as follows.

$$2.5\% \times (90 \ \mu L / (90+50) \ \mu L) = 1.6\%.$$

After the completion of the immunological reaction, the optical density (OD) of the transmitting light at 540 nm was measured by means of a plate reader. The difference between ODs at the Hb concentration of 0 ng/mL and 1000 ng/mL was determined and represented by ΔOD. The following Table 1 shows the results.

The solution viscosity shown in Table 1 is a viscosity (cP) measured at 40°C by means of B-type viscometer after said polymer has been added, in an amount of 2% of the mixture, to 50 mM sodium citrate solution containing 0.1% sodium azide and 0.01% Triton X100.

Table 1

| Water-Soluble Polymer | Solution Viscosity | ΔOD | (%) |
|---|---|---|---|
| no polymer (control) | - | 0.638 | 100 |
| (acrylamide)$_n$-(vinylpyrrolidone)$_n$ | 3.9 | 0.440 | 69 |
| polyvinylpyrrolidone PVPK17 | 3.9 | 0.493 | 77 |
| $(CH_2CH\text{-}COOCH_2CH(OH)CH_3)_{60}$ -$(CH_2CH\text{-}CONHCCH_2SO_3(CH_3)_2)_{40}$ | 4.4 | 0.614 | 96 |
| (acrylamide)$_{60}$-(N-vinylpyrrolidone)$_{38}$ -(methacryl alcohol)$_2$ | 4.8 | 0.333 | 52 |
| polyacrylamide | 5.6 | 0.571 | 89 |
| dimethylaminopropyl-acrylamide | 7.0 | 0.227 | 36 |
| polyvinylpyrrolidone PVPK90 | 8.0 | 0.089 | 14 |
| hydroxypropyl cellulose | 9.1 | 0.014 | 2 |
| sodium styrene-p-sulfonate | 9.4 | 0.055 | 9 |
| hydroxyethyl cellulose | 9.7 | 0.065 | 10 |
| methyl cellulose | 45.0 | 0.000 | 0 |

As shown in Table 1, in the control (no polymer), the transmission optical density at 540 nm decreased by agglutination with the existence of hemoglobin and the decrease (ΔOD) was about 0.64. Even when each of various polymers was co-existed in an amount of 1.6%, agglutination was observed as a decrease of $OD_{540}$ in some cases. Among the cases, only the polymers having a solution viscosity of 6 cP or less showed a sensitivity of about 50% or more as compared with the control.

Accordingly, even when a colloidal gold-labeled antibody is incorporated in a layer composed of a water-soluble polymer having a solution viscosity of 6 cP or less as the binder, agglutination in the layer construction of the element can be caused and the sensitivity can be expected to be almost equal to the conventional agglutination method in solution system. This can be expected by setting the final concentration of the polymer in the layer upon spotting a sample liquid to be not so different from 1.6%, which is the final concentration of the polymer in the aqueous solution capable of causing sensitive agglutination.

Example 2

Selection of Gelatin to which Colloidal Metal is incorporated

Experiments were conducted while using an immunological kit for detecting fecal occult hemoglobin "Immuno-Gold Hem" (product of Godo Shusei Co., Ltd., sold by Wako Pure Chemicals Industries Ltd.). Entirely the same operation was conducted as in Example 1 with the exception that each of various gelatins instead of various water-soluble polymers was added to the colloidal gold-labeled antibody solution so as to make the amount to be 2.5% by weight. The final concentration of the gelatin in the reaction mixture for the agglutination was 1.6% which is the same as that in Example 1. Table 2 shows the results. The solution viscosity shown in Table 2 is a viscosity (cP) measured at 40°C by means of B-type viscometer after said gelatin has been added, in an amount of 2% of the mixture, to mM sodium citrate solution (pH 6.0) containing 0.1% sodium azide and 0.01% Triton X-100.

Table 2

| Gelatin | Molecular Weight | Viscosity(cP) | $\Delta$OD |
|---|---|---|---|
| no gelatin(control) | - | - | 0.638 |
| gelatin | 20,000 | 56-61 | 0.611 |
| gelatin | 98,000 | 69-74 | 0.005 |
| gelatin | 98,000 | 73-75 | 0.007 |

As apparent from Table 2, in the case of gelatin having a molecular weight of 20,000, the decrease of $\Delta$OD was only a little and almost equal sensitivity was maintained as in the case of the control (no gelatin). On the other hand, the decrease of $\Delta$OD was remarkable in the case of gelatin having a high molecular weight of 98,000. Namely, it is observed a tendency that agglutination of colloidal gold-labeled antibody does not proceed and sufficient $\Delta$OD can not be attained when the gelatin having high molecular weight.

Accordingly, when a colloidal metal antibody is incorporated in a layer using a gelatin having a molecular weight of 20,000 or less as a binder, agglutination in the layer of the analysis element can be caused. The sensitivity can be expected to be almost equal to the conventional agglutination method in a solution system as long as the concentration of the gelatin in the layer upon spotting a sample liquid is set to be not so different from 1.6%, which is the concentration of the gelatin in the solution capable of causing the agglutination.

Example 3

Selection of Carboxymethylated Starch to which Colloidal Metal is incorporated

Similar to Examples 1 and 2, experiments were conducted while using an immunological kit for detecting fecal occult hemoglobin "Immuno-Gold Hem" (product of Godo Shusei Co., Ltd., sold by Wako Pure Chemicals Industries Ltd.). Entirely the same assay was conducted as in Example 1 with the exception that each of a carboxymethylated starch (CM-starch) as water-insoluble and water-swellable polymer instead of various water-soluble polymers in Example 1 was added to the colloidal gold-labeled antibody solution so as to make the concentration as shown in Table 3. Table 3 shows the results.

Table 3

| CM-Starch Concentration (v/w) | $\Delta$OD | % |
|---|---|---|
| 0.0% (control) | 0.638 | 100 |
| 0.5% | 0.606 | 95 |
| 1.0% | 0.543 | 85 |
| 1.5% | 0.585 | 92 |
| 2.0% | 0.464 | 73 |

As apparent from Table 3, in the case of carboxymethylated starch, sufficient $\Delta$OD value was obtained even when the starch was added in an amount of up to 2% to the colloidal gold-labeled antibody solution. Therefore, if the concentration of the carboxymethylated starch in the layer upon spotting a sample liquid is not more than or not so different from the final concentration in the solution-type agglutination, i.e., 1.28% (= 2.0% x (90 $\mu$L/(90+50) $\mu$L), a colloidal metal-labeled antibody can be incorporated in a layer of the element so that the agglutination takes place in the layer construction of the element. With such constitution, the sensitivity can be expected to be almost equal to the conventional solution-type agglutination method. At the dry analysis element in Example 5 mentioned below, a reagent layer was prepared by applying about 3% dispersion of the carboxymethylated starch, and the concentration of the carboxymethylated starch was calculated to be about 1.5% based on the liquid amount at spotting and the spread area. Although the solution-type agglutination was not examined at such a final concentration of 1.5%, it could be expected that the agglutination will take places without remarkable decrease of ΔOD even when the final concentration of the carboxymethylated starch was 1.5%. This expectation was supported by the result of Example 5 conducted according to a dry system.

Example 4

Preparation of Dry Analysis Element using Water-Soluble Polymer and Evaluation thereof

An aqueous solution of the following composition was coated on a colorless transparent smooth and flat polyethylene terephthalate film (support, thickness: 180 μm) undercoated with gelatin, followed by drying to form a reagent layer, whereby a dry analysis element for analyzing hemoglobin was prepared. The respective components had the coverage as set forth below.

| | |
|---|---|
| $(CH_2CH\text{-}COOCH_2CH(OH)CH_3)_{60}$ -$(CH_2CH\text{-}CONHCCH_2SO_3(CH_3)_2)_{40}$ | 7.5 g/m$^2$ |
| 50 mM sodium phosphate buffer (pH 7.0) | 242.3 g/m$^2$ |
| colloidal gold-labeled anti-human hemoglobin antibody | 200 mg/m$^2$ |

The thus prepared element was cut into rectangular chips of 12 x 13 mm size. The chips were severally encased with slide frames described in Unexamined Japanese Patent Publication No. 63452/1982 to prepare a dry slide 1 for analysis of hemoglobin according to the present example.

A human hemoglobin $A_0$ (Hb) (product of Exocell. INC) was diluted with 0.2 M ammonium chloride aqueous solution (pH 6.8) containing 6% polyethylene glycol 6000 to prepare a series of diluted solutions of 0, 100, 250, 500 and 1000 ng/mL. The series of diluted solutions was spotted onto the dry slide 1 in an amount of 10 μL each. After each slide was incubated at 37°C for 6 minutes, the reflection optical density at central wavelength of 540 nm was measured from PET support side. Upon measuring, a white plate made of Teflon (polytetrafluoroethylene) was placed at the reagent layer side as a reflecting plate which was used as a white background for color tone of the colloidal gold.

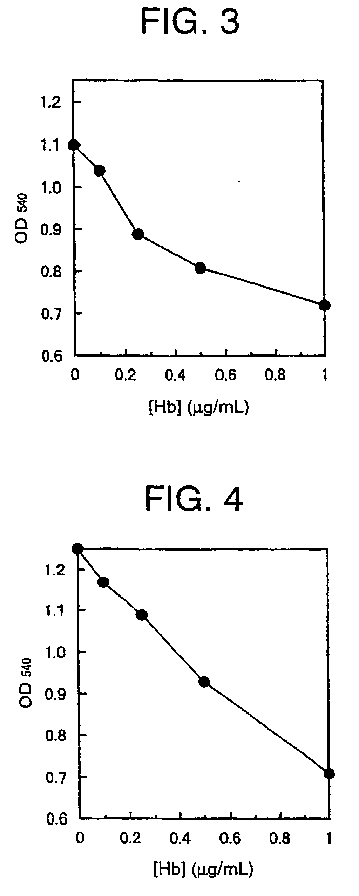

The results were shown in Fig. 3 as a calibration curve. As apparent from Fig. 3, the slide 1 comprising the reagent layer containing a water-soluble polymer exhibited a change of the reflection optical density $OD_{540}$ depending on the hemoglobin concentration in the samples. This fact showed that agglutination of hemoglobin (antigen) with colloidal gold-labeled antibody was caused in the reagent layer of the slide 1. In addition, it was confirmed that hemoglobin could be analyzed quantitatively with good sensitivity from a low concentration of 0.1 μg/mL. Furthermore, practical quantitative analysis was possible within only 6 minutes after the sample liquid had been spotted.

Example 5

Preparation of Dry Analysis Element using Insoluble Starch and Evaluation thereof

An aqueous solution of the following composition was coated on a colorless transparent smooth and flat polyethylene terephthalate film (support, thickness: 180 μm) undercoated with gelatin, followed by drying to form a reagent layer, whereby a dry analysis element for analyzing hemoglobin was prepared. The respective components had the coverage as set forth below.

| | |
|---|---|
| carboxymethylated starch | 7.5 g/m$^2$ |
| 50 mM sodium phosphate buffer (pH 7.0) | 242.3 g/m$^2$ |
| colloidal gold-labeled anti-human hemoglobin antibody | 200 mg/m$^2$ |

The prepared element was cut into rectangular chips of 12 x 13 mm size. The chips were encased with slide frames described in Unexamined Japanese Patent Publication No. 63452/1982 to prepare dry slide 2 for analysis of hemoglobin according to the present example.

A series of diluted solutions (0, 100, 250, 500 and 1000 ng/mL) prepared by diluting a human hemoglobin $A_0$ (Hb) (product of Exocell. INC) with 0.2 M ammonium chloride aqueous solution (pH 6.8) containing 6% polyethylene glycol 6000 was spotted onto the dry slide 2 in an amount of 10 μL each. After each slide was incubated at 37°C for 6 minutes, reflection optical density at central wavelength of 540 nm was measured from PET support side. Upon measuring, a white plate made of Teflon (polytetrafluoroethylene) was placed at the reagent layer side as a reflecting plate (white background).

Fig. 4 shows the results of the measurement with the slide 2 as a calibration curve. As apparent from Fig. 4, the slide 2 comprising the reagent layer where carboxymethylated starch, i.e., an insoluble starch was employed as a binder medium also exhibited that agglutination of hemoglobin (antigen) with colloidal gold-labeled antibody was caused in the reagent layer. In addition, it was confirmed that the calibration curve had a relatively good linearity over an all concentration range measured and therefore, hemoglobin could be analyzed more precisely with good sensitivity within a wide concentration range.

Example 6

Preparation of Dry Analysis Element laminated with Spreading Layer and Evaluation thereof

An aqueous solution of the following composition was coated on a colorless transparent smooth and flat polyethylene terephthalate (PET) film (support, thickness: 180 µm) undercoated with gelatin, followed by drying to form a reagent layer. The respective components had the coverage as set forth below.

| | |
|---|---|
| carboxymethylated starch | 7.5 g/m$^2$ |
| 50 mM sodium phosphate buffer (pH 7.0) | 242.3 g/m$^2$ |
| colloidal gold-labeled anti-human hemoglobin antibody | 200 mg/m$^2$ |

On the reagent layer was placed a silk screen, to which an adhesive for office job (starch paste) was applied by means of the squeeze method, followed by peeling off the screen to form mesh points of the adhesive on the reagent layer. Then, thereon was placed a white broad woven cloth made of a polyester which had been previously immersed in 10 mM phosphate buffer (pH 7.2; supplemented with 1.0% bovine serum albumin) at room temperature for 24 hours and dried. The cloth was pressed and adhered by slight pressure to form a spreading layer on the reagent layer, whereby a dry analysis element was prepared.

Then, the element was cut into rectangular chips of 12 x 13 mm size, and encased with slide frames described in Unexamined Japanese Patent Publication No. 63452/1982, whereby a dry slide 3 for analysis of hemoglobin according to the present example was prepared.

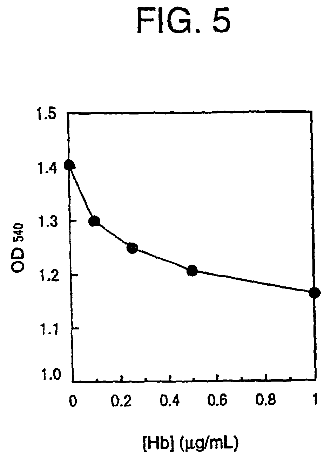

Onto the slide 3, the series of diluted hemoglobin solutions (0, 100, 250, 500 and 1000 ng/mL) which was the same as employed in Examples 4 and 5 was spotted in an amount of 20 µL each. After each slide was incubated at 37°C for 5 minutes, the reflection optical density at central wavelength of 540 nm was measured from PET support side. Since the spreading layer made of cloth also acted as a light reflective layer and a white background at measuring light, the reflection optical density was measured without placing a reflective plate at the reagent layer side. Fig. 5 shows the calibration curve obtained.

As apparent from Fig. 5, the slide 3 comprising the spreading layer on the reagent layer where carboxymethylated starch was employed as a binder medium also exhibited that hemoglobin could be quantitatively determined with good accuracy. Especially, the decrease of OD$_{540}$ was drastic at a low Hb concentration range. This fact shows that the slide 3 comprising the spreading layer allows more highly sensitive analysis and is suitable for quantitative determination of the analyte in lower concentration range as compared with the slides 1 and 2 comprising no spreading layer. In addition, it is confirmed that the slide 3 comprising the spreading layer enables to keep a liquid sample in the spreading layer upon spotting the liquid sample and thus operability is improved owing to no disturbance of a liquid flow at handling, at transportation to a measuring equipment, or at slide transportation within a measuring equipment.

Example 7

Storage Stability of Dry Analysis Element (1)

The storage stability of the dry analysis element (slide 2) obtained in Example 5 was examined. A dry analysis element is generally stable at 4°C for a duration of about 1 year. In this experiment, the elements were stored in a dry incubator set up at 35°C for 0, 1, 4, 7 days after preparation of the slides as an acceleration test.

As a comparative example, 250 µg/mL of colloidal gold-labeled anti-human hemoglobin antibody solution (50 mM sodium phosphate, pH 7.0) was prepared and used as a reagent for solution-type agglutination in the comparative example. The solution reagent of the comparative example was stored in an incubator of 35°C for 0, 1, 4, 7 days after the preparation in a similar manner of the slide 2.

100 ng/mL or 500 ng/mL of a human hemoglobin solution (human hemoglobin $A_0$ (Hb) (product of Exocell. INC): containing 6% polyethylene glycol 6000, 0.2 M ammonium chloride (pH 6.8)) was spotted, in an amount of 10 μL, onto each slide 2 after storing for the prescribed days. After each slide was incubated at 37°C for 6 minutes, the reflection optical density at 540 nm was measured from the support side. From the reflection optical density obtained, hemoglobin concentration was calculated based on the calibration curve made in Example 5 at the day when the slide 2 had been prepared.

As for the comparative example, 100 μL of the solution reagent stored in the prescribed days was mixed with 50 μL of 100 or 500 ng/mL of the human hemoglobin solution. After the reaction for 10 minutes, the transmission optical density at 540 nm was measured and hemoglobin concentration was calculated based on the calibration curve made by using standard solutions in advance.

As shown in Table 4, in the case of the solution reagent (the comparative example) to be employed for conventional solution-type colloidal gold agglutination, error of the calculated Hb concentration became larger during storage at 35°C with passage of days. The error reached to about 20% at fourth day and about 40% to 60% at seventh day from the beginning of the storage. On the other hand, the error was only 5 to 10% even at seventh day from the beginning of the storage in the case of the slide 2. As shown in the above, the dry analysis element according to the present invention is excellent in storage stability.

Table 4

| Comparison of Storage Stability | | | |
|---|---|---|---|
| Hb conc. (ng/mL) | Storage time (day) | Calculated Hb conc. (ng/mL) | |
| | | Slide 2 | Comparative |
| 100 | 0 | 100 | 100 |
| | 1 | 98 | 105 |
| | 4 | 103 | 120 |
| | 7 | 105 | 141 |
| 500 | 0 | 500 | 500 |
| | 1 | 510 | 550 |
| | 4 | 530 | 600 |
| | 7 | 560 | 850 |

Example 8

Storage Stability of Dry Analysis Element (2)

The storage stability of the dry analysis element (slide 3) obtained in Example 6 was examined according to the acceleration test, in the similar manner described in Example 7. 250 μg/mL of colloidal gold-labeled anti-human hemoglobin antibody solution (50 mM sodium phosphate, pH 7.0) used in Example 7 was also employed as a comparative example for the slide 3.

100 ng/mL or 500 ng/mL of a human hemoglobin solution (human hemoglobin $A_0$ (Hb) (product of Exocell. INC); containing 6% polyethylene glycol 6000, 0.2 M ammonium chloride (pH 6.8)) were spotted, in an amount of 20 μL, onto each slide 3 after storing for the prescribed days. After each slide was incubated at 37°C for 5 minutes, the reflection optical density at 540 nm was measured from the support side. From the reflection optical density obtained, hemoglobin concentration was calculated based on the calibration curve made in Example 6 at the day when the slide 3 had been prepared.

As for the comparative example, after storing the solution reagent for the prescribed days, assay was conducted according to the method entirely similar to that in Example 7, and then the transmission optical density at 540 nm was measured. Hemoglobin concentration was calculated based on the calibration curve made by using standard solutions in advance.

As shown in Table 5, in the slide 3, the dry analysis element according to the present invention is excellent in storage stability.

Table 5

| Comparison of Storage Stability | | | |
|---|---|---|---|
| Hb conc. (ng/mL) | Storage time (day) | Calculated Hb conc. (ng/ml) | |
| | | Slide 3 | Comparative |
| 100 | 0 | 100 | 100 |
| | 1 | 90 | 105 |
| | 4 | 105 | 120 |
| | 7 | 110 | 140 |
| 500 | 0 | 500 | 500 |
| | 1 | 510 | 550 |
| | 4 | 540 | 600 |
| | 7 | 570 | 850 |

As described above, the analysis method of the present invention utilizes a reagent layer comprising binder (s) containing any of a water-soluble polymer having a solution viscosity of 6 cP or less, a water-insoluble and water-swellable polymer, or gelatin having a molecular weight of 20,000 or less. Thereby, an agglutination of an analyte (e. g., antigen) with particles bearing an anti-analyte (e.g., colloidal gold-labeled antibody) can be caused in a reagent layer, one of layer structures of the dry analysis element. Accordingly, a speedy quantitative determination of the analyte by the agglutination can be conveniently attained with good sensitivity.

In addition, since the dry analysis element comprises a reagent layer using the binder(s), the element can be a medium of dry state, upon storage, to an extent not harmful to stability of the reagent composition to be used, and can also be a medium wetted by an aqueous test sample fed as an analyte antigen upon analysis to sufficiently cause agglutination of particles bearing an anti-analyte, whereby a highly sensitive analysis is made possible.

Furthermore, when particles bearing an anti-analyte are incorporated in the reagent layer or a layer thereon in advance, a highly sensitive dry analysis of the analyte can be conducted by simply spotting and feeding a liquid sample containing the analyte. As compared with the wet process agglutination using a conventional reagent solution, an extremely speedy and convenient analysis can be realized since preparation of the reagent at use and mixing operation are not necessary.

**Claims**

1. An agglutination assay method for quantitative determination of an analyte in an aqueous liquid sample using particles bearing an anti-analyte, the anti-analyte being capable of specifically binding to the analyte so as to cause agglutination of the particles, comprising:

    providing a reagent layer composed of at least one binder selected from the group consisting of:

       1) a water-soluble polymer, a solution of which has a viscosity of 6 cP or less;
       2) a water-insoluble and water-swellable polymer; and
       3) gelatin having a molecular weight of 20,000 or less;

    supplying said sample, together with said particles, to said reagent layer to cause the agglutination of said particles in said reagent layer; and
    measuring the extent of the agglutination of the particles in the reagent layer to determine the amount of the analyte in the sample.

2. The method according to claim 1, wherein said particle is a colloidal metal and the extent of the agglutination of the particles is detected from a change in color tone of the colloidal metals caused by the agglutination.

3. The method according to claim 2, wherein said colloidal metal is colloidal gold or colloidal silver.

4. The method according to claim 1, wherein said analyte is an antigen and said anti-analyte is an antibody.

5. An agglutination assay method for quantitative determination of an analyte in an aqueous liquid sample using particles bearing an anti-analyte, the anti-analyte being capable of specifically binding to the analyte so as to cause agglutination of the particles, comprising:

  providing a reagent layer containing said particles, the reagent layer being composed of at least one binder selected from the group consisting of:

    1) a water-soluble polymer, a solution of which has a viscosity of 6 cP or less;
    2) a water-insoluble and water-swellable polymer; and
    3) gelatin having a molecular weight of 20,000 or less;

  supplying said sample to the reagent layer to cause the agglutination of said particles in the reagent layer; and measuring the extent of the agglutination of the particles in the reagent layer to determine the amount of the analyte in the sample.

6. The method according to claim 5, wherein said particle is a colloidal metal and the extent of the agglutination of the particles is detected from a change in color tone of the colloidal metals caused by the agglutination.

7. The method according to claim 6, wherein said colloidal metal is colloidal gold or colloidal silver.

8. The method according to claim 6, wherein said analyte is an antigen and said anti-analyte is an antibody.

9. A dry analysis element for quantitative determination of an analyte in an aqueous liquid sample by measuring the extent of agglutination of particles bearing an anti-analyte, the anti-analyte being capable of specifically binding to the analyte so as to cause agglutination of the particles, comprising:

  a reagent layer composed of at least one binder selected from the group consisting of:

    1) a water-soluble polymer, a solution of which has a viscosity of 6 cP or less;
    2) a water-insoluble and water-swellable polymer; and
    3) gelatin having a molecular weight of 20,000 or less;

  whereby, when the sample is applied to the reagent layer together with said particles, the agglutination of said particles takes place in the reagent layer.

10. The dry analysis element according to claim 9, wherein said reagent layer contains said particles bearing the anti-analyte.

11. The dry analysis element according to claim 9, further comprising a spreading layer superposed on said reagent layer.

12. The dry analysis element according to claim 11, wherein said spreading layer contains said particles bearing the anti-analyte.

13. The dry analysis element according to claim 9, wherein said particle is a colloidal metal and the extent of the agglutination of the particles is detected from a change in color tone of the colloidal metals caused by the agglutination.

14. The element according to claim 13, wherein said colloidal metal is colloidal gold or colloidal silver.

15. The dry analysis element according to claim 9, wherein said analyte is an antigen and said anti-analyte is an antibody.

**Patentansprüche**

1. Agglutinationsassayverfahren zur quantitativen Bestimmung eines Analyten in einer wässrigen Flüssigprobe unter Verwendung von Partikeln, die einen Antianalyten umfassen, wobei der Antianalyt in der Lage ist, spezifisch an den Analyten zu binden, um so eine Agglutination der Partikel herbeizuführen, umfassend:

Bereitstellen einer Reagenzlage, die sich aus mindestens einem Bindemittel zusammensetzt, das ausgewählt ist aus der Gruppe bestehend aus:

1) einem wasserlöslichen Polymer, wobei eine Lösung daraus eine Viskosität von 6 cP oder weniger aufweist;
2) einem wasserunlöslichen und wasserquellbaren Polymer; und
3) Gelatine mit einem Molekulargewicht von 20.000 oder weniger;

Zuführen der Probe, zusammen mit den Partikeln, zu der Reagenzlage, um die Agglutination der Partikel in der Reagenzlage herbeizuführen; und
Messen des Ausmaßes der Agglutination der Partikel in der Reagenzlage, um die Menge des Analyten in der Probe zu bestimmen.

2. Verfahren nach Anspruch 1, wobei das Partikel ein kolloidales Metall ist und das Ausmaß der Agglutination der Partikel aufgrund der Veränderung des Farbtons der kolloidalen Metalle, die durch die Agglutination verursacht wird, bestimmt wird.

3. Verfahren nach Anspruch 2, wobei das kolloidale Metall kolloidales Gold oder kolloidales Silber ist.

4. Verfahren nach Anspruch 1, wobei der Analyt ein Antigen und der Antianalyt ein Antikörper ist.

5. Agglutinationsassayverfahren zur quantitativen Bestimmung eines Analyten in einer wässrigen Flüssigprobe unter Verwendung von Partikeln, die einen Antianalyten umfassen, wobei der Antianalyt in der Lage ist, spezifisch an den Analyten zu binden, um so eine Agglutination der Partikel herbeizuführen, umfassend:

Bereitstellen einer Reagenzlage, die die Partikel enthält, wobei sich die Reagenzlage aus mindestens einem Bindemittel zusammensetzt, das ausgewählt ist aus der Gruppe bestehend aus:

1) einem wasserlöslichen Polymer, wobei eine Lösung daraus eine Viskosität von 6 cP oder weniger aufweist;
2) einem wasserunlöslichen und wasserquellbaren Polymer; und
3) Gelatine mit einem Molekulargewicht von 20.000 oder weniger;

Zuführen der Probe zu der Reagenzlage, um die Agglutination der Partikel in der Reagenzlage herbeizuführen; und
Messen des Ausmaßes der Agglutination der Partikel in der Reagenzlage, um die Menge des Analyten in der Probe zu bestimmen.

6. Verfahren nach Anspruch 5, wobei das Partikel ein kolloidales Metall ist und das Ausmaß der Agglutination der Partikel aufgrund einer Veränderung im Farbton der kolloidalen Metalle, die durch die Agglutination verursacht wird, bestimmt wird.

7. Verfahren nach Anspruch 6, wobei das kolloidale Metall kolloidales Gold oder kolloidales Silber ist.

8. Verfahren nach Anspruch 6, wobei der Analyt ein Antigen und der Antianalyt ein Antikörper ist.

9. Trockenanalyseelement zur quantitativen Bestimmung eines Analyten in einer wässrigen Flüssigprobe durch Messen des Ausmaßes der Agglutination von Partikeln, die einen Antianalyten umfassen, wobei der Antianalyt in der Lage ist, spezifisch an den Analyten zu binden, um so eine Agglutination der Partikel herbeizuführen, umfassend:

eine Reagenzlage, die sich aus mindestens einem Bindemittel zusammensetzt, das ausgewählt ist aus der Gruppe bestehend aus:

1) einem wasserlöslichen Polymer, wobei eine Lösung daraus eine Viskosität von 6 cP oder weniger aufweist;
2) einem wasserunlöslichen und wasserquellbaren Polymer; und

3) Gelatine mit einem Molekulargewicht von 20.000 oder weniger;

wobei die Agglutination der Partikel in der Reagenzlage stattfindet, wenn die Probe zusammen mit den Partikeln auf die Reagenzlage aufgebracht wird.

10. Trockenanalyseelement nach Anspruch 9, wobei die Reagenzlage die Partikel, die den Antianalyten umfassen, enthält.

11. Trockenanalyseelement nach Anspruch 9, das außerdem eine Verteilungslage umfasst, die der Reagenzlage aufgelagert ist.

12. Trockenanalyseelement nach Anspruch 11, wobei die Verteilungslage die Partikel enthält, die den Antianalyten umfassen.

13. Trockenanalyseelement nach Anspruch 9, wobei das Partikel ein kolloidales Metall ist und das Ausmaß der Agglutination der Partikel aufgrund einer Veränderung im Farbton der kolloidalen Metalle, die durch die Agglutination verursacht wird, bestimmt wird.

14. Element nach Anspruch 13, wobei das kolloidale Metall kolloidales Gold oder kolloidales Silber ist.

15. Trockenanalyseelement nach Anspruch 9, wobei der Analyt ein Antigen und der Antianalyt ein Antikörper ist.

**Revendications**

1. Procédé de test d'agglutination pour la détermination quantitative d'un analyte dans un échantillon liquide aqueux au moyen de particules portant un anti-analyte, l'anti-analyte étant capable de se lier spécifiquement à l'analyte de manière à provoquer une agglutination des particules, comprenant :

la fourniture d'une couche de réactif composée d'au moins un liant choisi dans le groupe consistant en :

1) un polymère hydrosoluble, dont une solution a une viscosité de 6 cP ou moins ;
2) un polymère insoluble dans l'eau et gonflable dans l'eau ; et
3) de la gélatine ayant une masse moléculaire de 20 000 ou moins ;

l'apport dudit échantillon, en même temps que lesdites particules, à ladite couche de réactif pour provoquer l'agglutination desdites particules dans ladite couche de réactif ; et
la mesure de l'ampleur de l'agglutination des particules dans la couche de réactif pour déterminer la quantité d'analyte dans l'échantillon.

2. Procédé selon la revendication 1, où ladite particule est un métal colloïdal et l'ampleur de l'agglutination des particules est détectée d'après un changement de la teinte colorée des métaux colloïdaux provoqué par l'agglutination.

3. Procédé selon la revendication 2, où le métal colloïdal est l'or colloïdal ou l'argent colloïdal.

4. Procédé selon la revendication 1, où ledit analyte est un antigène et ledit anti-analyte est un anticorps.

5. Procédé de test d'agglutination pour la détermination quantitative d'un analyte dans un échantillon liquide aqueux au moyen de particules portant un anti-analyte, l'anti-analyte étant capable de se lier spécifiquement à l'analyte de manière à provoquer une agglutination des particules, comprenant :

la fourniture d'une couche de réactif contenant lesdites particules, la couche de réactif étant composée d'au moins un liant choisi dans le groupe consistant en :

1) un polymère hydrosoluble dont une solution a une viscosité de 6 cP ou moins ;
2) un polymère insoluble dans l'eau et gonflable dans l'eau ; et
3) de la gélatine ayant une masse moléculaire de 20 000 ou moins ;

l'apport dudit échantillon à la couche de réactif pour provoquer l'agglutination desdites particules dans la couche de réactif ; et

la mesure de l'ampleur de l'agglutination des particules dans la couche de réactif pour déterminer la quantité d'analyte dans l'échantillon.

6. Procédé selon la revendication 5, où ladite particule est un métal colloïdal et l'ampleur de l'agglutination des particules est détectée d'après un changement de la teinte colorée des métaux colloïdaux provoqué par l'agglutination.

7. Procédé selon la revendication 6, où ledit métal colloïdal est l'or colloïdal ou l'argent colloïdal.

8. Procédé selon la revendication 6, où ledit analyte est un antigène et ledit anti-analyte est un anticorps.

9. Elément d'analyse sec pour la détermination quantitative d'un analyte dans un échantillon liquide aqueux par mesure de l'ampleur de l'agglutination de particules portant un anti-analyte, l'anti-analyte étant capable de se lier spécifiquement à l'analyte de manière à provoquer une agglutination des particules, comprenant :

une couche de réactif composée d'au moins un liant choisi dans le groupe consistant en :

1) un polymère hydrosoluble dont une solution a une viscosité de 6 cP ou moins ;
2) un polymère insoluble dans l'eau et gonflable dans l'eau ; et
3) de la gélatine ayant une masse moléculaire de 20 000 ou moins;

de sorte que, quand l'échantillon est appliqué à la couche de réactif en même temps que lesdites particules, l'agglutination desdites particules a lieu dans la couche de réactif.

10. Elément d'analyse sec selon la revendication 9, où ladite couche de réactif contient lesdites particules portant l'anti-analyte.

11. Elément d'analyse sec selon la revendication 9 comprenant en outre une couche d'étalement située sur ladite couche de réactif.

12. Elément d'analyse sec selon la revendication 11, où ladite couche d'étalement contient lesdites particules portant l'anti-analyte.

13. Elément d'analyse sec selon la revendication 9, où ladite particule est un métal colloïdal et l'ampleur de l'agglutination des particules est détectée d'après un changement de teinte colorée des métaux colloïdaux provoqué par l'agglutination.

14. Elément selon la revendication 13, où ledit métal colloïdal est l'or colloïdal ou l'argent colloïdal.

15. Elément d'analyse sec selon la revendication 9, où ledit analyte est un antigène et ledit anti-analyte est un anticorps.

# FIG. 1

12

10

# FIG. 2

14

12

10

# FIG. 3

# FIG. 4

# FIG. 5